# EUROPÄISCHE PATENTSCHRIFT

(11) **EP 0 528 398 B1**
(45) Veröffentlichungstag und Bekanntmachung des Hinweises auf die Patenterteilung: **06.03.1996**
(21) Anmeldenummer: 92114020.8
(22) Anmeldetag: 17.08.1992
(51) Int. Cl.: A61B 17/32, A61B 10/00

(54) **Chirurgisches Instrument**
Surgical instrument
Instrument chirurgical

(30) Priorität: 16.08.1991 DE 4127166
(43) Veröffentlichungstag der Anmeldung: 24.02.1993
(73) Patentinhaber: Spitalny, Hans Henning, Dr. med., D-83209 Prien (DE)
(72) Erfinder: Spitalny, Hans Henning, Dr. med., D-83209 Prien (DE)

(56) Entgegenhaltungen:
- DE-A- 2 020 486
- FR-A- 2 068 926
- US-A- 3 913 566
- US-A- 4 022 191
- US-A- 4 913 143

## Beschreibung

Die Erfindung betrifft ein chirurgisches Instrument aus rostfreien Werkstoffen wie Edelstahl, Titan, Kunstoffen o.a. geeigneten Materialien, zur Entnahme und/oder Transplantation bzw. Implantation von Korium, Fett,Knorpel oder alloplastischem Material.

Üblicherweise wird Gewebe in entsprechender Größe dem Körper in der Weise entnommen, daß mit Hilfe eines Skalpells das entpsrechende Teil herausgetrennt wird. Fettgewebe wird häufig mit einer Kanüle entnommen, die mit einer Vakuumpumpe bzw. Spritze verbunden ist.

Diese Verfahren haben verschiedene Nachteile. Sie benötigen einen hohen Zeitaufwand bei der Entnahme des Gewebes und verursachen lange Narben, die eine Nachbehandlung erfordern.

Zerstörte Fettzellen führen zu Ölcysten mit nachfolgendem Gewebeabbau, der den therapeutischen Erfolg in Frage stellt.

Es sind aus der Literatur bereits Instrumente bekannt, die bei der Entnahme von Gewebeproben dienen.So betrifft die DE-OS 40 04 934 ein rohrförmiges, an einem Halter angesetztes Messer, dessen Umfangsflächen mit Markierungen versehen sind, um die Eindringtiefe des Messers in die Haut zu erkennen. Auch durch einen ringförmigen, auf das rohrförmige Messer aufsetzbaren Adapter verschiedener Länge kann die Eindringtiefe des Messers begrenzt werden.

Die DE-OS 21 03 910 betrifft einen am vorderen Rand zu einer Ringschneide angeschliffenen Hohlzylinder,der nach hinten in einen Griff übergeht, der an seinem der Ringscheide abgewandten Ende eine dreh-und abnehmbare Druckplatte trägt. Die Länge des Instruments ist so bemessen, daß die Druckplatte gegen die Handfläche des Operateurs drückt, wenn Daumen und Zeigefinger den Griff umfassen.

In der GB-PS 1 474 175 wird eine Vorrichtung zur Entnahme von Hautpfropfen aus der Kopfhaut zur Verpflanzung von Haaren beschrieben. Im Kopf des rohrförmigen, ggf. motorisch angetriebenen Messers befindet sich der Hautpfropfen nach der Entnahme.

Eine weitere Vorrichtung zum übertragen von Haaren auf der Kopfhaut von einer Stelle zur anderen wird in der US-PS 3 561 449 beschrieben. Diese Vorrichtung besteht im wesentlichen aus dem eigentlichen Schneideinstrument und dem Griff, wobei ausführlich erläutert wird, wie der Schneidkopf mit dem Griff verbunden ist.

Schließlich stellt die US-PS 3 913 566 ein zerlegbares Instrument dar, mit dem insbesondere Gewebeproben einem Körper entnommen werden, in dem Schneideteil des Instruments belassen,durch Abnehmen des Schneideteils und Einführen in den Griff untergebracht und zur weiteren Untersuchung versandt werden können.

Alle diese Instrumente dienen ausschließlich der Entnahme von Gewebeproben (Biopsie), die senkrecht zur Hautoberfläche erfolgt.

Es bestand daher die Aufgabe,ein chirurgisches Instrument zu schaffen, das es gestattet, auf einfache Weise ein ausreichend langes Stück Korium,Fett oder Knorpel einem entsprechenden Körperteil zu entnehmen und diese ggf. für Transplantationen zur Verfügung zu stellen.

Gelöst wird diese Aufgabe durch ein durch die Patentansprüche definiertes chirurgisches Instrument, das hervorragend zur Entnahme und/oder Transplantation von körpereigenem Gewebe geeignet ist. Das Instrument kann auch zur gezielten Implantation von Fremdmaterial verwendet werden.

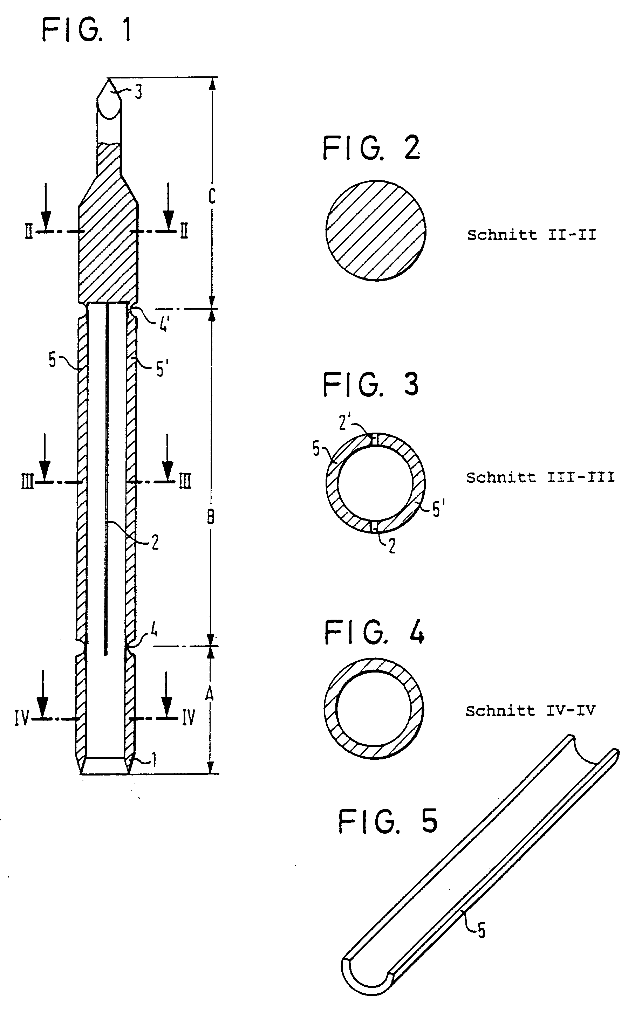

Das Instrument besteht im wesentlichen aus einer einseitig offenen Kanüle,die drei unterschiedliche,fest miteinander verbundene Teilbereiche aufweist (Fig.1) Der Teilbereich A ist rohrförmig und als Schneidevorrichtung ausgeildet. Sein offenes Ende (1) ist angeschliffen, sein entgegengesetztes Ende ist durch eine als Sollbruchstelle vorgesehene umlaufende Einkerbung (4) in der Wandung der Kanüle markiert. Das offene angeschliffene Ende besitzt bevorzugt eine mit einem Wellschliff ausgestattete Schneide (1).

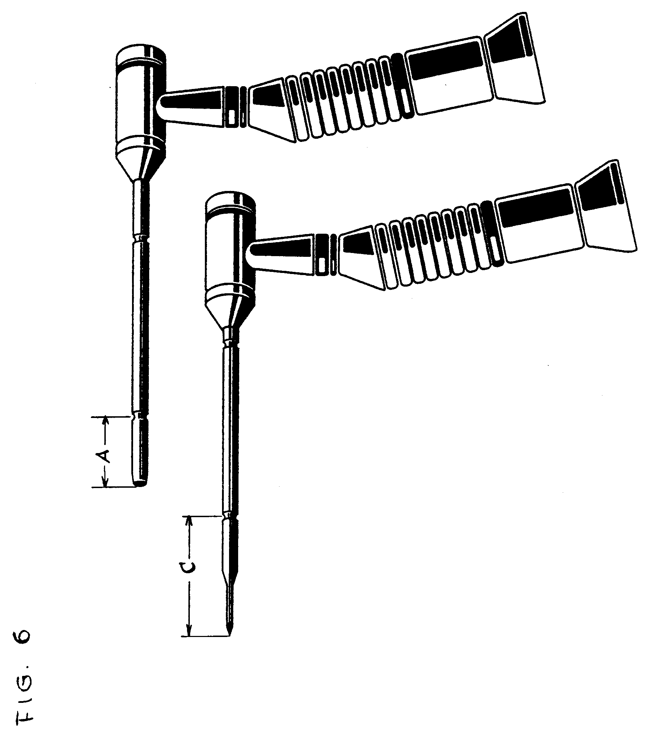

Mit Hilfe eines medizinischen Bohrgerätes, in das das Instrument mit seinem Teilbereich C oder A eingesetzt werden kann, wird es in Rotation versetzt und ist damit für den Schneid-bzw. Bohrvorgang bereit, die Oberhaut an der Entnahmestelle zu durchtrennen (Fig.6). Die Kanüle wird parallel zur Hautoberfläche rotierend vorgeschoben.

Der mittlere Teilbereich B des Instruments besteht aus einer oder mehreren mit den Bereichen A und C fest verbundenen Schalen. Dieser Teilbereich B entsteht dadurch, daß das dem Teilbereich B ebenfalls durch eine als Sollbruchstelle vorgesehene Einkerbung (4') markiert ist und eine oder mehrere Trennstellen (2,2') parallel zur Kanülenachse in der Wandung (5,5') der Kanüle aufweist. Bevorzugt weist der Bereich B zwei auf gegenüberliegenden Seiten befindliche Trennstellen 2 und 2') auf, so daß zwei Halbschalen (5 und 5') gebildet werden. Die Trennstelle zwischen den Halbschalen ( 5 und 5') ist 0 bis 0,5 mm breit. Zwischen den Schalen befindet sich nach Beendigung des Vorschubs des Instruments das zylinderförmig herausgetrennte Gewebe. Nach Abbrechen der Teilbereiche A und C (nachdem das Instrument entsprechend in die Haut ein- und an einer anderen Stelle wieder hinausgeführt wurde) an den Stellan der Einkerbungen 4 und 4' verbleibt der Teilbereich B in Form von einer oder mehrerer voneinander getrennter Schalen 5 und 5' unter der Hautoberfläche des zu behandelnden Körperteils. Das zwischen den Schalen befindliche Gewebe verbleibt durch radiales geringfügiges Anheben und axiales Herausziehen der einzelnen Schalensegmente in der eingebrachten Position und wird lediglich noch durch Trimmen geglättet.

Der Bereich C des Instruments ist aus Vollmaterial und verjüngt sich zur einer pyramiden- oder kegelförmig angeschliffenen Nadel (3).

Die Übergänge der Teilbereiche A nach B und B nach C sind mit umlaufenden Einkerbungen 4 und 4' in der Wandung der Kanüle markiert. Diese kennzeichnen dadurch das Ende des Schneid-, des mittleren und des aus Vollmaterial bestehenden Teilbereich C des Instruments. Diese Einkerbungen stellen rechteckige, keilförmige, quadratische oder halbrunde Ausnehmungen in der Wandung der Kanüle dar und bestimmen infolge der Querschnittsreduzierung der Wandung Sollbruchstellen.

Die Figuren 2 bis 4 geben die Querschnitte bei den in Figur 1 dargestellten Stellen II-II (Teilbereich C), III-III (Teilbereich B) und IV-IV (Teilbereich A) des Instruments an. Figur 5 stellt eine Halbschale des Instruments nach Abbrechen der Teilbereiche A und C dar.

Die einzelnen Bereiche des Instruments haben folgende Längenabmessungen: Teilbereich A 5 bis 20 mm, Teilbereich B 20 bis 150 mm, Teilbereich C 10 bis 50 mm.

Bevorzugt wird ein Instrument verwendet, bei dem die Teilbereiche A 2cm, B 10 cm und C 2 cm lang sind.

Die lichte Weite der Kanüle kann je nach vorgesehenem Verwendungszweck und Art der zu entnehmenden bzw. zu übertragenden Gewebepartie 1 bis 20 mm betragen. Im allgemeinen ist für Hauttransplantationen eine lichte Weite der Kanüle von 1,5 mm ausreichend.

Die Breite der parallel zur Kanülenachse verlaufenden Trennstellen, welche die Schalen des Mittelteils B bilden. können 0 bis 0,5 mm betragen. Die zu wählende Spaltbreite hängt von der Anwendung des Instruments ab.

Neben der Entnahme und Transplantation von körpereigenem Gewebe, wie z.B. Korium, Fett oder Knorpel kann das Instrument auch Zur Implantation von Fremdmaterial, also alloplastischem Material wie z.B. Collagen oder Silikon, verwendet werden. Durch entsprechende Einführung des Instruments unter die zu behandelnden Körperstellen kann das Material damit genau plaziert werden.

Vorzugsweise wird das Instrument aus rostfreien Werkstoffen wie z.B. Edelstahl, Titan, Glas oder Kunststoff hergestellt.

## Patentansprüche

1. Chirurgisches Instrument zur Entnahme und/oder Transplantation bzw. Implantation von Korium, Fett,Knorpel oder alloplastischem Material, bestehend aus einem einseitig offenen kanülenförmigen Gehäuse mit den Teilbereichen A,B und C wobei
- Teilbereich A rohrförmig und als Schneidvorrichtung ausgebildet ist, dessen offenes Ende (1) angeschliffen und dessen anderes Ende durch eine als Sollbruchstelle vorgesehene umlaufende Einkerbung (4) in der Wandung der Kanüle markiert ist,
- Teilbereich B ebenfalls rohrförmig ausgebildet ist und das dem Teilbereich A abgewandten Ende des Teilbereichs B durch eine als Sollbruchstelle vorgesehene Einkerbung (4') markiert ist und eine oder mehrere Trennstellen (2,2') parallel zur Kanülenachse in der Wandung (5,5') der Kanüle derart aufweist,daß nach Abbrechen der Teilbereiche A und C der Teilbereich B in Form von einer oder mehrerer voneinander getrennter Schalen vorliegt.
- und Teilbereich C aus Vollmaterial besteht und zu einer scharf angeschliffenen Nadel (3) ausläuft.

2. Instrument nach Anspruch 1, dadurch gekennzeichnet, daß Bereich A 5 bis 20 mm, Bereich B 20 bis 150 mm, Bereich C 10 bis 50 mm lang ist.

3. Instrument nach den Ansprüchen 1 und 2, dadurch gekennzeichnet, daß die lichte Weite der Teilbereiche A und B der Kanüle 1 bis 20 mm beträgt.

4. Instrument nach den Ansprüchen 1 bis 3, dadurch gekennzeichnet, daß die Schneidvorrichtung A einen Wellschliff besitzt.

5. Instrument nach Ansprüchen 1 bis 4, dadurch gekennzeichnet, daß der Bereich B zwei Trennstellen (2,2') besitzt.

6. Instrument nach Ansprüchen 1 bis 5, dadurch gekennzeichnet, daß die Breite der Trennstellen (2,2') zwischen den Schalen (5,5') des Bereichs B 0 bis 0,5mm beträgt.

7. Instrument nach Ansprüchen 1 bis 6, dadurch gekennzeichnet, daß die Einkerbungen (4 und 4') keilförmige Querschnittsverengungen in der Wand der Kanüle darstellen.

8. Instrument nach Ansprüchen 1 bis 7, dadurch gekennzeichnet, daß die Einkerbungen (4 und 4') keilförmige Querschnittsverengungen in der Wand der Kanüle darstellen.

9. Instrument nach Ansprüchen 1 bis 8, dadurch gekennzeichnet daß es aus rostfreien Werkstoffen besteht.

10. Instrument nach Anspruch 9, dadurch gekennzeichnet, daß es aus Edelstahl besteht.

## Claims

1. A surgical instrument for the removal, transplantation or implantation of corium, fat or cartilage. It consists of a cannula open on one end comprising the sections A, B and C.
- Section A is tubular in shape and designed in the form of a scalpel device. Its open end (1) is sharpened and the other end is marked by an annular notch (4) in the wall of the cannula.
- Section B is likewise tubular in shape as a result of the end of section B that is situated opposite Section A marked with another annular notch (4') and comprising two or more seperating seams (2,2') extending over the entire length of Section B parallel to the longitudinal axis of the cannula in the walls thereof (5,5'). After Sections A and C are broken off at annular notches Section B remains in the form of one or more shells seperated from one another (FIG. 3 and 5).
- Section C of the instrument is constructed of solid material tapered to form a pyramidal or conical sharpened needle (3).

2. A surgical instrument of claim 1, wherein the length of the various sections are as follows: section A - 5 to 20 mm, section B - 20 to 150 mm, section C - 10 to 50 mm.

3. A surgical instrument of claims 1 and 2, wherein the inside diameter of sections A and B may be from 1 to 20 mm.

4. A surgical instrument of claims 1 to 3, wherein the scalpel end is cylindrical sharpened.

5. A surgical instrument of claims 1 to 4, wherein section B has two seperation seams.

6. A surgical instrument of claims 1 to 5, wherein the width of the seperation seams that form the shells of section B may be 0 to 0.5 mm.

7. A surgical instrument of claims 1 to 6, wherein the wall of the cannula is marked by annular notches (4 and 4').

8. A surgical instrument of claims 1 to 7, wherein the wall of the cannula is marked by wedge-shaped notches (4 and 4').

9. A surgical instrument of claims 1 to 8 made of stainless material.

10. A surgical instrument of claim 9 made of stainless steel.

## Revendications

1. Instrument chirurgical pour l'extraction., la transplantation, et l'implantation de chorion, graisse, cartilage, ou matériaux plastiques anallergiques, constitué paritellement en forme de canule ouverte à une extrémité, et se décomposant en trois parties A, B et C :
- La partie A, en forme de tube, constitue un instrument de coupe, dont une extrémité est ouverte et aiguisée, et dont l'autre extrémité es repérée par l'encoche circulaire ,,4" sur le corps de la canule.
- La partie B, elle aussi en forme de tube, commence à l'extrémité de la partie A, et se termine à son autre extrémité au niveau de l'encoche circulaire ,,4'" dans la paroi de la canule. La partie B comprend aussi une ou plusieurs fentes verticales dans la (les) poroi (s) (f et 5') de la canule, et constitue, après séparation d'avec les parties A et C, un tube (schéma 3) ou plusieurs pièces en forme de gouttiére (schéma 5).
- La partie C, massive, se termine par une aiguille pointue et aiguisée.

2. Les dimensions de l'instrument décrit au paragraphe 1 sont les suivantes :
- Longuéur de la partie A : de 5 à 20 mm
- Longuéur de la partie B: de 20 à 150 mm
- Longuéur de la partie C : de 10 à 50 mm

3. Dans l'instrument décrit aux paragraphes 1 et 2, la largeur des parties A et B de la canule est comprise entre 1 et 20 mm.

4. Dans l'instrument décrit aux paragraphes 1 à 3, le dispositif de coupe A comprend un cylindre poli.

5. Dans l'instrument décrit aux paragraphes 1 à 4, la partie B est munie de deux fentes verticale (2 et 2').

6. Dans l'instrument décrit aux paragraphes 1 à 5, et dans la partie B de l'instrument, la largeur des fentes verticales 2 et 2'entre les parties 5 et 5' est comprise entre 0 et 0,5 mm.

7. Dans l'instrument décrit aux paragraphes 1 à 6, lex encoches de forme semi-circulaires 4 et 4'forment une dépression dans les parois de la canule.

8. Dans l'instrument décrit aux paragraphes 1 à 7 : lex encoches en forme de coin 4 et 4'forment une dépression dans les parois de la canule.

9. L'instrument décrit aux paragraphes 1 à 8 est fabriqué en matériau synthétique inoxydable.

10. L'instrument décrit au paragraphe 9 est fabrique en acier inoxydable.
